# EUROPÄISCHE PATENTSCHRIFT

(11) **EP 1 758 546 B1**
(45) Veröffentlichungstag und Bekanntmachung des Hinweises auf die Patenterteilung: **05.09.2007**
(21) Anmeldenummer: 05746189.9
(22) Anmeldetag: 30.04.2005
(51) Int. Cl.: A61Q 5/06, A61K 8/49, A61K 8/34, A61K 8/39

(54) **VERWENDUNG VON SPEZIELLEN POLYOLEN ZUR KRISTALLISATIONSINHIBIERUNG VON ONIUMALDEHYDEN BZW. -KETONEN**
USE OF SPECIAL POLYOLS FOR INHIBITING THE CRYSTALLIZATION OF ONIUM ALDEHYDES AND ONIUM KETONES
UTILISATION DE POLYOLS SPECIAUX POUR INHIBER LA CRISTALLISATION D'ALDEHYDES D'ONIUM ET DE CETONES D'ONIUM

(30) Priorität: 19.05.2004 DE 102004025282
(43) Veröffentlichungstag der Anmeldung: 07.03.2007
(73) Patentinhaber: Henkel Kommanditgesellschaft auf Aktien, 40589 Düsseldorf (DE)
(72) Erfinder: OBERKOBUSCH, Doris, 40591 Düsseldorf (DE); GROSS, Wibke, 40549 Düsseldorf (DE); MOCH, Melanie, 41542 Dormagen (DE)
(86) Internationale Anmeldenummer: PCT/EP2005/004702
(87) Internationale Veröffentlichungsnummer: WO 2005/115322

(56) Entgegenhaltungen:
- WO-A-01/47483
- US-A1- 2002 002 748
- US-A1- 2004 226 109
- US-B1- 6 371 993

## Beschreibung

Die Erfindung betrifft die Verwendung von speziellen Polyolen zur Kristallisationsinhibierung von Oniumaldehyden bzw. -ketonen in wässrigen Zusammensetzungen, eine Zusammensetzung, enthaltend eine Kombination aus Oniumaldehyden bzw. ketonen, Polyol und Wasser, die Verwendung dieser Kombination in Mitteln zur Färbung von keratinhaltigen Fasern und ein Kit-of-parts, mit Hilfe dessen die erfindungsgemäße Zusammensetzung für die Durchführung des Verfahrens bereitgestellt wird.

Für das Färben von keratinhaltigen Fasern, z. B. Haaren, Wolle oder Pelzen, kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Kuppler- und Entwicklerkomponenten werden auch als Oxidationsfarbstoffvorprodukte bezeichnet.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazolon-5, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin und 2,5,6-Triamino-4-hydroxypyrimidin.

Als Kupplerkomponenten werden in der Regel m-Phenylendiaminderivate, Naphthole, Resorcin und Resorcinderivate, Pyrazolone und m-Aminophenole verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 2,4-Diaminophenoxyethanol, 1-Phenyl-3-methyl-pyrazolon-5, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin und 5-Methylresorcin.

Bezüglich weiterer üblicher Farbstoffkomponenten wird ausdrücklich auf die Reihe "Dermatology", herausgeben von Ch. Culnan, H. Maibach, Verlag Marcel Dekker lnc., New York, Basel, 1986, Bd. 7, Ch. Zviak, The Science of Hair Care, Kap. 7, Seiten 248 - 250 (Direktziehende Farbstoffe) und Kap. 8, Seiten 264 - 267 (Oxidationsfarbstoffe) sowie das "Europäische Inventar der Kosmetikrohstoffe", 1996, herausgegeben von der Europäischen Kommission, erhältlich in Diskettenform vom Bundesverband der deutschen Industrie- und Handelsunternehmen für Arzneimittel, Reformwaren und Körperpflegemittel e.V., Mannheim, Bezug genommen.

Mit Oxidationsfarbstoffen lassen sich zwar intensive Färbungen mit guten Echtheitseigenschaften erzielen, die Entwicklung der Farbe geschieht jedoch im Allgemeinen unter Zusatz chemischer Oxidationsmittel wie z.B. H₂O₂, was in einigen Fällen Schädigungen der Faser zur Folge haben kann. Desweiteren können einige Oxidationsfarbstoffvorprodukte bzw. bestimmte Mischungen von Oxidationsfarbstoffvorprodukten bisweilen bei Personen mit empfindlicher Haut sensibilisierend wirken. Direktziehende Farbstoffe werden unter schonenderen Bedingungen appliziert, ihr Nachteil liegt jedoch darin, daß die Färbungen häufig nur über unzureichende Echtheitseigenschaften verfügen.

Eine Möglichkeit, Färbungen mit guten Echtheitseigenschaften ohne den Einfluß von zusätzlichen chemischen Oxidationsmitteln zu erzielen ist die Anwendung von sogenannten Oxofärbemitteln. Oxofärbemittel bieten die Möglichkeit keratinhaltige Fasern zu färben, mittels Verwendung einer Kombination aus
Komponente A): Verbindungen, die eine reaktive Carbonylgruppe enthalten, mit
Komponente B): Verbindungen, ausgewählt aus (a) CH-aciden Verbindungen, (b) Verbindungen mit primärer oder sekundärer Aminogruppe, ausgewählt aus primären oder sekundären aromatischen Aminen, stickstoffhaltigen heterozyklischen Verbindungen und aromatischen Hydroxyverbindungen, (c) Aminosäuren, (d) aus 2 bis 9 Aminosäuren aufgebauten Oligopeptiden.

Die entsprechende Färbemethode (im folgenden Oxofärbung genannt) wird beispielweise in den Druckschriften WO-A1-99/18916, WO-A1-00/38638, WO-A1-01/34106 und WO-A1-01/47483 beschrieben. Die resultierenden Färbungen besitzen teilweise Farbechtheiten auf der keratinhaltigen Faser, die mit denen der Oxidationsfärbung vergleichbar sind. Das mit der schonenden Oxofärbung erzielbare Nuancenspektrum ist sehr breit und die erhaltene Färbung weist oftmals eine akzeptable Brillanz und Farbtiefe auf. Die vorgenannten Komponenten A und B, im weiteren als Oxofarbstoffvorprodukte bezeichnet, sind im allgemeinen selbst keine Farbstoffe, und eignen sich daher jede für sich genommen allein nicht zur Färbung keratinhaltiger Fasern. In Kombination bilden sie in einem nichtoxidativen Prozess Farbstoffe aus. Unter Verbindungen der Komponente B können allerdings auch entsprechende Oxidationsfarbstoffvorprodukte vom Entwickler- und/oder Kupplertyp mit oder ohne Einsatz eines Oxidationsmittels Verwendung finden. Somit läßt sich die Methode der Oxofärbung ohne weiteres mit dem oxidativen Färbesystem kombinieren.

Im Rahmen der Oxofärbung werden als Komponente A reaktive Carbonylverbindungen eingesetzt, die insbesondere nach Reaktion mit einer Komponente B den eigentlichen Farbstoff im Haar ausbilden. Bevorzugte reaktive Carbonylverbindungen sind Aldehyde und Ketone, in denen die reaktive Carbonylgruppe entweder als Carbonylgruppe vorliegt oder derart derivatisiert bzw. maskiert ist, daß die Reaktivität des Kohlenstoffatoms der derivatisierten Carbonylgruppe gegenüber den Verbindungen der Komponente B stets vorhanden ist. Diese Derivate sind bevorzugt Additionsverbindungen
a) von Aminen und deren Derivate unter Bildung von Iminen oder Oximen als Additionsverbindung
b) von Alkoholen unter Bildung von Acetalen oder Ketalen als Additionsverbindung
c) von Wasser unter Bildung von Hydraten als Additionsverbindung (Komponente A leitet sich in diesem Fall c) von einem Aldehyd ab) an das Kohlenstoffatom der Carbonylgruppe der reaktiven Carbonylverbindung.

Als hervorragende reaktive Carbonylverbindungen haben sich Oniumaldehyde bzw. - ketone erwiesen. Allerdings besitzen diese speziellen reaktiven Carbonylverbindungen ein ausgeprägtes Kristallisationsverhalten in wässrigen Medien und lassen sich daher nicht dauerhaft in ausreichender Konzentration in einen wasserhaltigen Träger einarbeiten. Die dauerhafte Einarbeitung der Oxofarbstoffvorprodukte in den kosmetischen Träger ist jedoch zur Bereitstellung eines anwendungsfähigen Färbemittels sehr vorteilhaft. In den bisher bekannten kosmetischen Formulierungen agglomeriert das gelöste Oniumaldehyd bzw. -keton nach einigen Stunden bzw. Tagen der Lagerung und fällt anschließend als gegebenenfalls kristalliner Feststoff aus dem kosmetischen Träger aus. Unter "Kristallisation" wird erfindungsgemäß das Ausfallen des agglomerierten amorphen oder kristallinen Feststoffs verstanden.

Verwendet man die durch Kristallisation gebildeten inhomogenen Färbemittel, kann eine ungleichmäßige Färbung auftreten oder sich die Anwendungsdauer erhöhen. Insbesondere bei einer Absonderung feiner Kristalle der Oniumaldehyde bzw. -ketone aus dem Träger haben Tests mit Probanden ergeben, dass während der Anwendung ein unerwünschtes Kratzen wahrgenommen wird. Es besteht somit zur Vermeidung dieser Nachteile ein Bedarf nach Zusammensetzungen, in welchen Oniumaldehyde bzw. - ketone dauerhaft eingearbeitet sind.

Aufgabe der vorliegenden Erfindung ist es, Färbemittel für Keratinfasern, insbesondere menschliche Haare, bereitzustellen, in denen die Kristallisation der Oniumaldehyde bzw. -ketone inhibiert wird und die Formulierungen somit lagerstabil sind. Darüber hinaus dürfen diese Färbemittel kein oder lediglich ein sehr geringes Sensibilisierungspotential und Anfärbevermögen der Haut aufweisen, sollen jedoch das Haar gleichmäßig über die gesamte Länge intensiv und farbecht färben.

Unter keratinhaltigen Fasern sind Wolle, Pelze, Federn und insbesondere menschliche Haare zu verstehen. Die erfindungsgemäßen Färbemittel können prinzipiell aber auch zum Färben anderer Naturfasern, wie z.B. Baumwolle, Jute, Sisal, Leinen oder Seide, modifizierter Naturfasern, wie z.B. Regeneratcellulose, Nitro-, Alkyl- oder Hydroxyalkyl- oder Acetylcellulose und synthetischer Fasern, wie z.B. Polyamid-, Polyacrylnitril-, Polyurethan- und Polyesterfasern verwendet werden.

Es wurde überraschenderweise gefunden, dass diese Aufgabe durch Zusatz einer bestimmten Menge eines linearen oder verzweigten, aliphatischen Polyols gelöst wird.

Gegenstand der vorliegenden Erfindung ist demgemäß die Verwendung von 2 bis 50 Gew.-%, bevorzugt 5 bis 30 Gew.-%, besonders bevorzugt 5 bis 20 Gew.-%, jeweils bezogen auf das Gewicht der wässrigen Zusammensetzung, eines linearen oder verzweigten, aliphatischen Polyols zur Kristallisationsinhibierung von Oniumaldehyden und -ketonen gemäß Formel I und deren Derivate 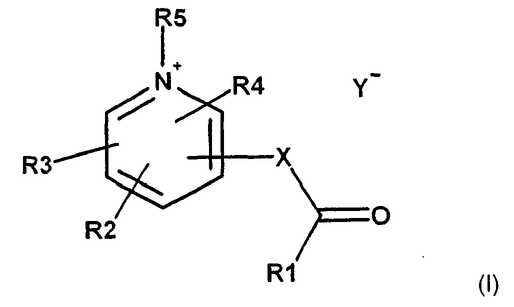 worin
- R1 für ein Wasserstoffatom, eine (C₁-C₆)-Alkylgruppe, eine Arylgruppe oder eine Heteroarylgruppe steht,
- R2, R3 und R4 jeweils unabhängig voneinander ein Wasserstoffatom, ein Halogenatom, eine (C₁-C₆)-Alkylgruppe, (C₁-C₆)-Alkoxygruppe, (C₁-C₆)-Hydroxyalkylgruppe, Hydroxy-(C₁-C₆)-alkoxygruppe, Hydroxygruppe, Nitrogruppe, Arylgruppe, Trifluormethylgruppe, Aminogruppe, die durch (C₁-C₆)-Alkylgruppen substituiert sein kann, oder (C₁-C₆)-Acylgruppe bedeuten, wobei auch zwei der Reste gemeinsam einen ankondensierten Benzolring bilden können,
- R5 für eine (C₁-C₆)-Alkylgruppe, Arylgruppe, Aryl-(C₁-C₆)-alkylgruppe oder Heteroarylgruppe steht,
- X für eine direkte Bindung oder eine ggf. substituierte Vinylen- oder Phenylengruppe steht, und
- Halogenid, Benzolsulfonat, p-Toluolsulfonat, Methansulfonat, Trifluormethansulfonat, Perchlorat, Sulfat, Hydrogensulfat, Tetrachlorzinkat oder N-Oxid des Heterocyclus bedeutet,

in wässrigen Zusammensetzungen.

Die Verbindungen gemäß Formel I liegen bevorzugt bei 40°C, insbesondere bei 20°C, als Feststoff vor.

Die Derivate der Oniumaldehyde bzw. -ketone sind Additionsverbindungen
a) von Aminen und deren Derivaten unter Bildung von Iminen oder Oximen als Additionsverbindung
b) von Alkoholen unter Bildung von Acetalen oder Ketalen als Additionsverbindung
c) von Wasser unter Bildung von Hydraten als Additionsverbindung (Komponente A leitet sich in diesem Fall c) von einem Aldehyd ab)
an das Kohlenstoffatom der Carbonylgruppe der reaktiven Carbonylverbindung. Insbesondere sind Hydrate als Derivate gemäß c) bevorzugt.

Beispiele für die als Substituenten im Rahmen dieser Anmeldung genannten (C₁-C₆)-Alkylreste sind die Gruppen Methyl, Ethyl, Propyl, lsopropyl, n-Butyl, tert-Butyl, n-Pentyl, neo-Pentyl und n-Hexyl. Ethyl und Methyl sind bevorzugte Alkylreste. Beispiele für bevorzugte C₂-C₆-Alkenylreste sind Vinyl, Allyl und Butenyl. Erfindungsgemäß bevorzugte C₁-C₆-Alkoxyreste sind beispielsweise eine Methoxy- oder eine Ethoxygruppe. Weiterhin können als bevorzugte Beispiele für eine C₁-C₆-Hydroxyalkylgruppe eine Hydroxymethyl-, eine 2-Hydroxyethyl-, eine 2-Hydroxypropyl, eine 3-Hydroxypropyl- oder eine 4-Hydroxybutylgruppe genannt werden. Eine 2-Hydroxyethylgruppe ist besonders bevorzugt. Beispiele für eine bevorzugte C₂-C₆-Polyhydroxyalkylgruppe sind die 1,2-Dihydroxyethylgruppe und die 2,3-Dihydroxypropylgruppe. Beispiele für eine (C₁-C₆)-Acylgruppe sind Acetyl, Propanoyl und Butanoyl. Bevorzugte Hydroxy-(C₁-C₆)-alkoxygruppen sind 2-Hydroxyethoxy, 2-Hydroxypropoxy und 3-Hydroxypropoxy. Bevorzugte Arylgruppen sind Phenyl, Naphthyl und Biphenyl. Beispiele für eine Heteroarylgruppe sind Pyrrolidyl, 2-Furyl, 2-Thienyl, 4-Pyridyl, 3-Pyridyl, 2-Pyridyl, Triazolyl und 1-Imidazolyl. Beispiele für eine Heterozyklus-C₁₋₄-alkylgruppe sind Pyrrolidino-(C₁₋₄)-alkyl, Piperidino-(C₁₋₄)-alkyl, Morpholino-(C₁₋₄)-alkyl, 2-Furyl-(C₁₋₄)-alkyl, 2-Thienyl-(C₁₋₄)-alkyl, 4-Pyridyl-(C₁₋₄)-alkyl, 3-Pyridyl-(C₁₋₄)-alkyl, 2-Pyridyl-(C₁₋₄)-alkyl, Triazolyl-(C₁₋₄)-alkyl und 1-Imidazolyl-(C₁₋₄)-alkyl. Bevorzugte Aryl-(C₁-C₆)-alkylgruppen sind Benzyl, 2-Phenylethyl, 5-Phenylpropyl, wobei Benzyl besonders bevorzugt ist. Beispiele für Halogenatome sind F-, Cl-, oder Br-Atome, wobei Cl-Atome ganz besonders bevorzugt sind. Die weiteren verwendeten Begriffe leiten sich erfindungsgemäß von den hier gegebenen Definitionen ab.

Die Verbindungen mit der Formel I werden üblicherweise in einer Menge von jeweils 1 bis 30 mmol, insbesondere 2 bis 15 mmol, jeweils bezogen auf 100 g der gesamten Zusammensetzung, eingesetzt.

Die Oniumaldehyde bzw. -ketone werden bevorzugt ausgewählt aus der Gruppe bestehend aus den Benzolsulfonaten, p-Toluolsulfonaten, Methansulfonaten, Trifluormethansulfonaten, Perchloraten, Sulfaten, Chloriden, Bromiden, Jodiden und/oder Tetrachlorzinkaten von 4-Formyl-1-methylpyridinium, 3-Formyl-1-methylpyridinium, 2-Formyl-1-methylpyridinium, 4-Formyl-1-ethylpyridinium, 2-Formyl-1-ethylpyridinium, 4-Formyl-1-benzylpyridinium, 2-Formyl-1-benzylpyridinium, 4-Formyl-1,2-dimethylpyridinium, 4-Formyl-1,3-dimethylpyridinium, 4-Formyl-1-methylchinolinium, 2-Formyl-1-methylchinolinium, 4-(2-Formylvinyl)-1-methylchinolinium, 4-Acetyl-1-methyl-pyridinium, 2-Acetyl-1-methylpyridinium, 4-Acetyl-1-chinolinium, 4-Acetyl-1-methylchinolinium und 4-(2-Formylvinyl)-1-methylpyridinium, 2,6-Dichlor-4-formyl-1-methylpryridinium, 2,6-Diphenyl-4-formyl-1-methylpyridinium, 4-Benzoyl-1-methylpyridinium, 4-Propionyl-1-methylpyridinium, 2-Oximomethyl-1-methylpyridinium, 4-Pyridincarboxaldehyd-N-oxid und N-Methylpyridoxal.

Gemäß einer besonders bevorzugten Ausführungsform werden die Oniumaldehyde bzw. -ketone ausgewählt aus Formyl-1-methylchinolinium-Verbindungen mit der folgenden Formel II, 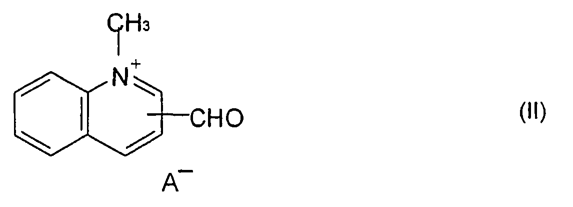 worin die Formylgruppe CHO in 2- oder 4-Position gebunden ist und A⁻ steht für ein p-Toluolsulfonat-, Methansulfonat-, Trifluormethansulfonat-, Methylsulfat- oder Benzolsulfonat-Ion. Das bevorzugte Anion ist gemäß dieser Ausführungsform das p-Toluolsulfonat-Ion.

Die Oniumaldehyde und -ketone gemäß Formel I bzw. ll besitzen eine Löslichkeit von maximal 1 mmol, insbesondere maximal 0.5 mmol, in 100 g Wasser bei einer Temperatur von 20 °C.

4-Formyl-1-methyl-chinolinium p-Toluolsulfonat und 2-Formyl-1-methyl-chinolinium p-Toluolsulfonat sind ganz besonders bevorzugte Verbindungen gemäß Formeln I und II.

Unter dem Begriff Polyol werden erfindungsgemäß Verbindungen definiert, welche zwei oder mehr Hydroxygruppen besitzen. Die erfindungsgemäßen Polyole besitzen bevorzugt eine Löslichkeit von mindestens 0.5 g, insbesondere von 2 g, in 100 g Wasser bei 20 °C.

Bevorzugte Vertreter der erfindungsgemäß verwendbaren Polyole werden aus einer Gruppe ausgewählt, die gebildet wird aus HO-(CH₂CH₂O)ₙ-H mit n = 1 bis 14, Dipropylenglykol, Tripropylenglykol, Glycerin, Diglycerin, 1,2-Butandiol, 1,4-Butandiol, 1,5-Pentandiol, 1,2-Pentandiol, 2-Methyl-2,4-pentandiol, 4-Methyl-2-pentanol, 1,2,6-Hexantriol und 2-Ethyl-1,3-hexandiol. Ein besonders bevorzugtes Polyol ist Ethylenglykol (HO-CH₂CH₂-OH).

Wässrige Zusammensetzungen enthalten bevorzugt mindestens 20 Gew.% Wasser, besonders bevorzugt mindestens 40 Gew.% Wasser, bezogen auf das Gewicht der Zusammensetzung.

Es ist bevorzugt, die linearen oder verzweigten, aliphatischen Polyole in Kombination mit mindestens einem Polymer, ausgewählt aus der Gruppe, die gebildet wird aus anionischen, amphoteren, nichtionischen und kationischen Polymeren, insbesondere ausgewählt aus kationischen und/oder nichtionischen Polymeren, zur Inhibierung der Kristallisation der Oniumaldehyde bzw. -ketone zu verwenden. Dabei kann es wiederum bevorzugt sein, wenn die oben genannten Polymertypen von Polysacchariden und dessen Derivaten abgeleitet sind.

Bei den verwendbaren anionischen Polymeren, handelt es sich um ein anionische Polymere, welche Carboxylat- und/oder Sulfonatgruppen und/oder Phosphatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure.

Ein Beispiel für ein anionisches Polymer mit einer Phosphatgruppe ist das unter dem Handelsnamen Structure^{®} Zea vertriebene Polysaccharidderivat (NATIONAL STARCH) (INCI-Bezeichnung: Hydroxypropyl Starch Phosphate).

Weitere verwendbare anionische Polymere, enthalten als alleiniges oder Co-Monomer 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegen kann.

Besonders bevorzugt ist das Homopolymer der 2-Acrylamido-2-methylpropansulfonsäure, das beispielsweise unter der Bezeichnung Rheothik^{®}11-80 im Handel erhältlich ist.

Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester.

Weitere anionische Copolymere sind Acrylsäure-Acrylamid-Copolymere sowie insbesondere Polyacrylamidcopolymere mit Sulfonsäuregruppen-haltigen Monomeren. Ein solches anionisches Copolymer besteht bevorzugt aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylen-bisacrylamid zum Einsatz kommen. Ein solches Polymer ist in dem Handelsprodukt Sepigel^{®}305 der Firma SEPPIC enthalten. Die Verwendung dieses Compounds, das neben der Polymerkomponente eine Kohlenwasserstoffmischung (C₁₃-C₁₄-Isoparaffin) und einen nichtionogenen Emulgator (Laureth-7) enthält, hat sich im Rahmen der erfindungsgemäßen Lehre als besonders vorteilhaft erwiesen.

Auch die unter der Bezeichnung Simulgel^{®}600 als Compound mit Isohexadecan und Polysorbat-80 vertriebenen Natriumacryloyldimethyltaurat-Copolymere haben sich als erfindungsgemäß besonders wirksam erwiesen.

Ebenfalls bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichen Carbopol^{®} im Handel erhältlich.

Copolymere aus Maleinsäureanhydrid und Methylvinylether, insbesondere solche mit Vernetzungen, sind ebenfalls farberhaltende Polymere. Ein mit 1,9-Decadiene vernetztes Maleinsäure-Methylvinylether-Copolymer ist unter der Bezeichnung Stabileze^{®} QM im Handel erhältlich.

Weiterhin können als Polymere amphotere Polymere eingesetzt werden. Unter dem Begriff amphotere Polymere werden sowohl solche Polymere, die im Molekül sowohl freie Aminogruppen als auch freie -COOH- oder SO₃H-Gruppen enthalten und zur Ausbildung innerer Salze befähigt sind, als auch zwitterionische Polymere, die im Molekül quartäre Ammoniumgruppen und -COO⁻- oder -SO₃⁻-Gruppen enthalten, und solche Polymere zusammengefaßt, die -COOH- oder SO₃H-Gruppe und quartäre Ammoniumgruppen enthalten.

Ein Beispiel für ein erfindungsgemäß einsetzbares Amphopolymer ist das unter der Bezeichnung Amphomer^{®} erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.

Weitere erfindungsgemäß einsetzbare amphotere Polymere sind die in der britischen Offenlegungsschrift 2 104 091, der europäischen Offenlegungsschrift 47 714, der europäischen Offenlegungsschrift 217 274, der europäischen Offenlegungsschrift 283 817 und der deutschen Offenlegungsschrift 28 17 369 genannten Verbindungen.

Bevorzugt eingesetzte amphotere Polymere sind solche Polymerisate, die sich im wesentlichen zusammensetzen aus
(a) Monomeren mit quartären Ammoniumgruppen der allgemeinen Formel P1,

   R¹-CH=CR²-CO-Z-(CₙH₂ₙ)-N⁽⁺⁾R³R⁴R⁵ A⁽⁻⁾ (P1)

   in der R¹ und R² unabhängig voneinander stehen für Wasserstoff oder eine Methylgruppe und R³, R⁴ und R⁵ unabhängig voneinander für Alkylgruppen mit 1 bis 4 Kohlenstoff-Atomen, Z eine NH-Gruppe oder ein Sauerstoffatom, n eine ganze Zahl von 2 bis 5 und A⁽⁻⁾ das Anion einer organischen oder anorganischen Säure ist
   und
(b) monomeren Carbonsäuren der allgemeinen Formel P2,

   R¹-CH=CR²-COOH (P2)

   in denen R¹ und R² unabhängig voneinander Wasserstoff oder Methylgruppen sind.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Bezüglich der Einzelheiten der Herstellung dieser Polymerisate wird ausdrücklich auf den Inhalt der deutschen Offenlegungsschrift 39 29 973 Bezug genommen. Ganz besonders bevorzugt sind solche Polymerisate, bei denen Monomere der Formel P1 eingesetzt werden, bei denen R³, R⁴ und R⁵ Methylgruppen sind, Z eine NH-Gruppe und A⁽⁻⁾ ein Halogenid-, Methoxysulfat- oder Ethoxysulfat-Ion ist; Acrylamidopropyl-trimethyl-ammoniumchlorid ist ein besonders bevorzugtes Monomeres gemäß Formel P1. Als Monomeres der Formel P2 für die genannten Polymerisate wird bevorzugt Acrylsäure verwendet.

Unter kationischen Polymeren sind Polymere zu verstehen, welche in der Haupt- und/oder Seitenkette Gruppen aufweisen, welche "temporär" oder "permanent" kationisch sein kann. Als "permanent kationisch" werden erfindungsgemäß solche Polymere bezeichnet, die unabhängig vom pH-Wert der Mittel eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C₁₋₄-Kohlenwasserstoffgruppe an eine aus Acrylsäure, Methacrylsäure oder deren Derivaten aufgebaute Polymerhauptkette gebunden sind, haben sich als besonders geeignet erwiesen. Homopolymere der allgemeinen Formel P3, 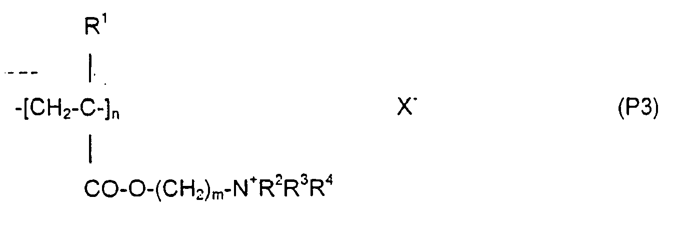 in der R¹=-H oder-CH₃ ist, R², R³ und R⁴ unabhängig voneinander ausgewählt sind aus C₁₋₄-Alkyl-, -Alkenyl- oder -Hydroxyalkylgruppen, m = 1, 2, 3 oder 4, n eine natürliche Zahl und X⁻ ein physiologisch verträgliches organisches oder anorganisches Anion ist, sowie Copolymere, bestehend im wesentlichen aus den in Formel P3 aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gemäß Formel P3 gilt:
- R¹ steht für eine Methylgruppe
- R², R³ und R⁴ stehen für Methylgruppen
- m hat den Wert 2.

Als physiologisch verträgliches Gegenion X⁻ gemäß Formel P3 kommen beispielsweise Halogenidionen, Sulfationen, Phosphationen, Methosulfationen sowie organische lonen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylen-bisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Copolymere mit Monomereinheiten gemäß Formel P3 enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare^{®} SC 92 erhältlich.

Weitere bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate,
- kationische Alkylpolyglycoside gemäß der DE-PS 44 13 686,
- kationiserter Honig, beispielsweise das Handelsprodukt Honeyquat^{®} 50,
- kationische Guar-Derivate, wie insbesondere die unter den Handelsnamen Cosmedia^{®}Guar (COGNIS), N-Hance® 3000, N-Hance® 3196, N-Hance® 3205, N-Hance^{®} 3215 (alle AQUALON) und Jaguar^{®} (RHODIA) vertriebenen Produkte,
- Polysiloxane mit quaternären Gruppen, wie beispielsweise die im Handel erhältlichen Produkte Q2-7224 (Hersteller: Dow Corning; ein stabilisiertes Trimethylsilylamodimethicon), Dow Corning^{®} 929 Emulsion (enthaltend ein hydroxyl-aminomodifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80),
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905 und HM 552 angeboten werden.
- quaternierter Polyvinylalkohol,
sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft^{®} LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind.

Weitere erfindungsgemäße kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen^{®} CMF, Hydagen^{®} HCMF, Kytamer^{®} PC und Chitolam^{®} NB/101 im Handel frei verfügbar sind. Chitosane sind deacetylierte Chitine, die in unterschiedlichen Deacetylierungsgraden und unterschiedlichen Abbaugraden (Molekulargewichten) im Handel erhältlich sind. Ihre Herstellung ist z.B. in DE 44 40 625 A1 und in DE 1 95 03 465 A1 beschrieben.

Besonders gut geeignete Chitosane weisen einen Deacetylierungsgrad von wenigstens 80 % und ein Molekulargewicht von 5 · 10⁵ bis 5 · 10⁶ (g/mol) auf.

Zur Herstellung erfindungsgemäßer Zubereitungen muß das Chitosan in die Salzform überführt werden. Dies kann durch Auflösen in verdünnten wäßrigen Säuren erfolgen. Als Säuren sind sowohl Mineralsäuren wie z.B. Salzsäure, Schwefelsäure und Phosphorsäure als auch organische Säuren, z.B. niedermolekulare Carbonsäuren, Polycarbonsäuren und Hydroxycarbonsäuren geeignet. Weiterhin können auch höhermolekulare Alkylsulfonsäuren oder Alkylschwefelsäuren oder Organophosphorsäuren verwendet werden, soweit diese die erforderliche physiologische Verträglichkeit aufweisen. Geeignete Säuren zur Überführung des Chitosans in die Salzform sind z.B. Essigsäure, Glycolsäure, Weinsäure, Apfelsäure, Citronensäure, Milchsäure, 2-Pyrrolidinon-5-carbonsäure, Benzoesäure oder Salicylsäure. Bevorzugt werden niedermolekulare Hydroxycarbonsäuren wie z.B. Glycolsäure oder Milchsäure verwendet.

Besonders bevorzugt verwendete nichtionogene Polymere werden ausgewählt aus:
- Vinylpyrrolidon/Vinylester-Copolymere, wie sie beispielsweise unter dem Warenzeichen Luviskol^{®} (BASF) vertrieben werden. Luviskol^{®} VA 64 und Luviskol^{®} VA 73, jeweils Vinylpyrrolidon/Vinylacetat-Copolymere, sind ebenfalls bevorzugte nichtionische Polymere.
- Cellulose und Derivate davon, insbesondere Celluloseether, wie Hydroxypropylcellulose, Hydroxyethylcellulose und Methylhydroxypropylcellulose, wie sie beispielsweise unter den Warenzeichen Klucel^{®} (INCI-Bezeichnung: Hydroxypropylcellulose), Culminal^{®} (lNCl-Bezeichnung: Hydroxypropylcellulose) und Benecel^{®} (INCI-Bezeichnung: Hydroxypropyl Methylcellulose) (alle AQUALON) sowie Natrosol^{®} (AQUALON), insbesondere Natrosol^{®} 250 HR (INCI-Bezeichnung: Hydroxyethylcellulose) und Natrosol^{®} Plus 330 CS (INCI-Bezeichnung: Cetylhydroxyethylcellulose) vertrieben werden.
- Guar und Derivate davon, wie sie beispielsweise unter der Warenzeichen N-Hance® HP-40 (AQUALON) vertrieben werden
- Stärke-Fraktionen und Derivate davon, wie beispielsweise Amylose, Amylopektin und Dextrine, die z.B. unter dem Handelsnamen Amaze^{®} und Structure^{®} Solanance (NATIONAL STARCH) erhältlich sind
- Schellack
- Polyvinylpyrrolidone, wie sie beispielsweise unter der Bezeichnung Luviskol^{®} (BASF) vertrieben werden.
- Agar-Agar, Alginate, Xanthan-Gum, Gummi arabicum, Karaya-Gummi, Johannisbrotkernmehl, Leinsamengummen, Dextrane,.

Es ist besonders bevorzugt, solche nichtionischen Polymere zu verwenden, welche sich von einem Polysaccharid ableiten, wie Cellulose und seine Derivate, Stärke-Fraktionen und deren Derivate, Guar und seine Derivate.

Die Polymere werden erfindungsgemäß bevorzugt in Mengen von 0,05 bis 5 Gew.-%, bezogen auf die gesamte Zusammensetzung, eingesetzt. Mengen von 0,25 bis 2 Gew.% sind besonders bevorzugt.

Ein zweiter Gegenstand der Erfindung ist eine Zusammensetzung, enthaltend eine Kombination aus Komponente
(i) mindestens eines Oniumaldehyds und -ketons der folgenden Formel I oder deren Derivaten 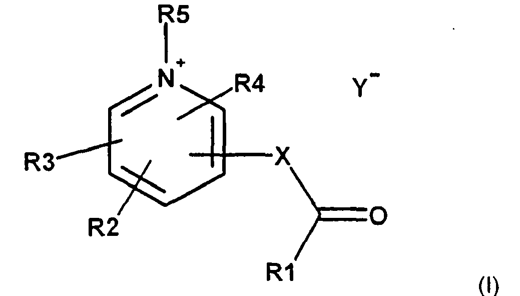 worin R1, R2, R3, R4, R5, X und Y⁻ wie oben definiert sind.
   mit Komponente
(ii) 2 bis 50 Gew.-%, bezogen auf das Gewicht der gesamten Zusammensetzung, mindestens eines linearen oder verzweigten, aliphatischen Polyols und
(iii) Wasser.

Es können diejenigen bevorzugten Oniumaldehyde bzw. -ketone gegebenenfalls in den bevorzugten Mengenbereichen in den erfindungsgemäßen Zusammensetzungen eingesetzt werden, die bereits im ersten Gegenstand der Erfindung beschrieben wurden.

Es können diejenigen bevorzugten linearen oder verzweigten, aliphatischen Polyole gegebenenfalls in den bevorzugten Mengenbereichen in den erfindungsgemäßen Zusammensetzungen eingesetzt werden, die bereits im ersten Gegenstand der Erfindung beschrieben wurden.

Bevorzugt enthält die erfindungsgemäße Zusammensetzung mindestens ein Polymer. Es können diejenigen bevorzugten Polymere gegebenenfalls in den bevorzugten Mengenbereichen in den erfindungsgemäßen Zusammensetzungen eingesetzt werden, die bereits im ersten Gegenstand der Erfindung beschrieben wurden.

Färbungen mit erhöhter Brillanz und verbesserten Echtheitseigenschaften (Lichtechtheit, Waschechtheit, Reibechtheit) über einen weiten Nuancenbereich von gelb, orange, rot, violett, braun, bis hin zu blauschwarz und schwarz werden erzielt, wenn die erfindungsgemäß eingesetzten Verbindungen mit der Formel I gemeinsam mit mindestens einer weiteren farbgebenden Komponente B, ausgewählt aus CH-aciden Verbindungen und/oder Verbindungen mit primärer oder sekundärer Aminogruppe, ausgewählt aus der Gruppe, die gebildet wird aus primären oder sekundären aromatischen Aminen, stickstoffhaltigen heterocyclischen Verbindungen und aromatischen Hydroxyverbindungen, verwendet werden. Zu diesem Zweck ist es bevorzugt, dass das zur Anwendung kommende Färbemittel, d.h. die Anwendungsmischung gemäß erfindungsgemäßem Färbeverfahren des dritten Gegenstandes dieser Erfindung, mindestens eine dieser zusätzlichen färbenden Komponente enthält.

Als CH-acide werden im allgemeinen solche Verbindungen angesehen, die ein an ein aliphatisches Kohlenstoffatom gebundenes Wasserstoffatom tragen, wobei durch eine Aktivierung der entsprechenden Kohlenstoff-Wasserstoff-Bindung dieses Wasserstoffatom mit einer Base abstrahiert werden kann.

Die CH-aciden Verbindungen sind bevorzugt ausgewählt aus der Gruppe bestehend aus 1,2,3,3-Tetramethyl-3H-indoliumiodid, 1,2,3,3-Tetramethyl-3H-indolium-p-toluolsulfonat, 1,2,3,3-Tetramethyl-3H-indolium-methansulfonat, 1,3,3-Trimethyl-2-methylenindolin (Fischersche Base), 2,3-Dimethyl-benzothiazoliumiodid, 2,3-Dimethyl-benzothiazoliump-toluolsulfonat, 2,3-Dimethyl-naphtho[1,2-d]thiazolium-p-toluolsulfonat, 3-Ethyl-2-methyl-naphtho[1,2-d]thiazolium-p-toluolsulfonat, Rhodanin, Rhodanin-3-essigsäure, 1,4-Dimethylchinolinium-iodid, 1,2-Dimethylchinolinium-iodid, Barbitursäure, Thiobarbitursäure, 1,3-Dimethylthiobarbitursäure, 1,3-Diethylthiobarbitursäure, 1,3-Diethylbarbitursäure, Oxindol, 3-Indoxylacetat, 2-Cumaranon, 5-Hydroxy-2-cumaranon, 6-Hydroxy-2-cumaranon, 3-Methyl-1-phenyl-pyrazolin-5-on, Indan-1,2-dion, Indan-1,3-dion, Indan-1-on, Benzoylacetonitril, 3-Dicyanmethylenindan-1-on, 2-Amino-4-imino-1,3-thiazolin-hydrochlorid, 5,5-Dimethylcyclohexan-1,3-dion, 2H-1,4-Benzoxazin-4H-3-on, 3-Ethyl-2-methyl-benzoxazoliumiodid, 3-Ethyl-2-methyl-benzothiazoliumiodid, 1-Ethyl-4-methyl-chinoliniumiodid, 1-Ethyl-2-methylchinoliniumiodid, 1,2,3-Trimethylchinoxaliniumiodid, 3-Ethyl-2-methyl-benzoxazolium-p-toluolsulfonat, 3-Ethyl-2-methyl-benzothiazolium-p-toluolsulfonat, 1-Ethyl-4-methyl-chinolinium-p-toluolsulfonat, 1-Ethyl-2-methylchinolinium-p-toluolsulfonat, 1,2,3-Trimethylchinoxalinium-p-toluolsulfonat 1,2-Dihydro-1,3,4,6-tetramethyl-2-oxo-pyrimidinium-chlorid, 1,2-Dihydro-1,3-diethyl-4,6-dimethyl-2-oxo-pyrimidinium-chlorid, 1,2-Dihydro-1,3-dipropyl-4,6-dimethyl-2-oxo-pyrimidinium-chlorid, 1,2-Dihydro-1,3,4,6-tetramethyl-2-oxo-pyrimidinium-hydrogensulfat, 1,2-Dihydro-1,3-diethyl-4,6-dimethyl-2-oxo-pyrimidinium-hydrogensulfat, 1,2-Dihydro-1,3-dipropyl-4,6-dimethyl-2-oxo-pyrimidinium-hydrogensulfat, 1,2-Dihydro-1,3,4-trimethyl-2-oxo-pyrimidinium-chlorid, 1,2-Dihydro-1,3,4-trimethyl-2-oxo-pyrimidinium-hydrogensulfat, 1,2-Dihydro-1,3-diethyl-4-methyl-2-oxo-pyrimidinium-chlorid, 1,2-Dihydro- 1,3-diethyl-4-methyl-2-oxo-pyrimidinium-hydrogensulfat, 1,2-Dihydro-1,3-dipropyl-4-methyl-2-oxo-pyrimidinium-chlorid, 1,2-Dihydro-1,3-dipropyl-4-methyl-2-oxo-pyrimidinium-hydrogensulfat, 1,2-Dihydro-1, 3,4,6-tetramethyl-2-thioxo-pyrimidinium-chlorid, 1,2-Dihydro-1,3-diethyl-4,6-dimethyl-2-thioxo-pyrimidinium-chlorid, 1,2-Dihydro-1,3-dipropyl-4,6-dimethyl-2-thioxo-pyrimidinium-chlorid, 1,2-Dihydro-1,3,4,6-tetramethyl-2-thioxo-pyrimidinium-hydrogensulfat, 1,2-Dihydro-1,3-dipropyl-4,6-dimethyl-2-thioxo-pyrimidinium-hydrogensulfat, 1,2-Dihydro-1,3,4-trimethyl-2-thioxo-pyrimidinium-chlorid, 1,2-Dihydro-1,3,4-trimethyl-2-thioxo-pyrimidinium-hydrogensulfat, 1,2-Dihydro-1,3-diethyl-4-methyl-2-thioxo-pyrimidinium-chlorid, 1,2-Dihydro-1,3-diethyl-4-methyl-2-thioxo-pyrimidinium-hydrogensulfat, 1,2-Dihydro-1,3-dipropyl-4-methyl-2-thioxo-pyrimidinium-chlorid und 1,2-Dihydro-1,3-dipropyl-4-methyl-2-thioxo-pyrimidinium-hydrogensulfat.

Die primären und sekundären aromatischen Amine sind bevorzugt ausgewählt aus der Gruppe bestehend aus N,N-Dimethyl-p-phenylendiamin, N,N-Diethyl-p-phenylendiamin, N-(2-Hydroxyethyl)-N-ethyl-p-phenylendiamin, N,N-Bis-(2-hydroxyethyl)-p-phenylendiamin, N-(2-Methoxyethyl)-p-phenylendiamin, 2,3-Dichlor-p-phenylendiamin, 2,4-Dichlor-p-phenylendiamin, 2,5-Dichlor-p-phenylendiamin, 2-Chlor-p-phenylendiamin, 2,5-Dihydroxy-4-morpholinoanilin, 2-Aminophenol, 3-Aminophenol, 4-Aminophenol, 2-Aminomethyl-4-aminophenol, 2-Hydroxymethyl-4-aminophenol, o-Phenylendiamin, m-Phenylendiamin, p-Phenylendiamin, 2,5-Diaminotoluol, 2,5,-Diaminophenol, 2,5-Diaminoanisol, 2,5-Diaminophenethol, 4-Amino-3-methylphenol, 2-(2,5-Diaminophenyl)-ethanol, 2,4-Diaminophenoxyethanol, 2-(2,5-Diaminophenoxy)-ethanol, 3-Amino-4-(2-hydroxyethyloxy)phenol, 3,4-Methylendioxyphenol, 3,4-Methylendioxyanilin, 3-Amino-2,4-dichlorphenol, 4-Methylaminophenol, 2-Methyl-5-aminophenol, 3-Methyl-4-aminophenol, 2-Methyl-5-(2-hydroxyethylamino)phenol, 3-Amino-2-chlor-6-methylphenol, 2-Methyl-5-amino-4-chlorphenol, 5-(2-Hydroxyethylamino)-4-methoxy-2-methylphenol, 4-Amino-2-hydroxymethylphenol, 2-(Diethylaminomethyl)-4-aminophenol, 4-Amino-1-hydroxy-2-(2-hydroxyethylaminomethyl)-benzol, 1-Hydroxy-2-amino-5-methyl-benzol, 1-Hydroxy-2-amino-6-methyl-benzol, 2-Amino-5-acetamido-phenol, 1,3-Dimethyl-2,5-diaminobenzol, 5-(3-Hydroxypropylamino-)2-methylphenol, 5-Amino-4-methoxy-2-methylphenol, N,N-Dimethyl-3-aminophenol, N-Cyclopentyl-3-aminophenol, 5-Amino-4-fluor-2-methylphenol, 2,4-Diamino-5-fluortoluol, 2,4-Diamino-5-(2-hydroxyethoxy)-toluol, 2,4-Diamino-5-methylphenetol, 3,5-Diamino-2-methoxy-1-methylbenzol, 2-Amino-4-(2-hydroxyethylamino)-anisol, 2,6-Bis-(2-hydroxyethylamino)-1-methylbenzol, 1,3-Diamino-2,4-dimethoxybenzol, 3,5-Diamino-2-methoxy-toluol, 2-Aminobenzoesäure, 3-Aminobenzoesäure, 4-Aminobenzoesäure, 2-Aminophenylessigsäure, 3-Aminophenylessigsäure, 4-Aminophenylessigsäure, 2,3-Diaminobenzoesäure, 2,4-Diaminobenzoesäure, 2,5-Diaminobenzoesäure, 3,4-Diaminobenzoesäure 3,5-Diaminobenzoesäure, 4-Aminosalicylsäure, 5-Aminosalicylsäure, 3-Amino-4-hydroxybenzoesäure, 4-Amino-3-hydroxy-benzoesäure, 2-Aminobenzolsulfonsäure, 3-Aminobenzolsulfonsäure, 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-Triaminobenzol, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogallol, 1-(2-Hydroxy-5-aminobenzyl)-2-imidazolidinon, 4-Amino-2-((4-[(5-amino-2-hydroxyphenyl)methyl]-piperazinyl)methyl)phenol, 3,5-Diamino-4-hydroxybrenzcatechin, 1,4-Bis-(4-aminophenyl)-1,4-diazacycloheptan, aromatische Nitrile, wie 2-Amino-4-hydroxybenzonitril, 4-Amino-2-hydroxybenzonitril, 4-Aminobenzonitril, 2,4-Diaminobenzonitril, Nitrogruppen-haltige Aminoverbindungen, wie 3-Amino-6-methylamino-2-nitro-pyridin, Pikraminsäure, [8-[(4-Amino-2-nitrophenyl)-azo]-7-hydroxy-naphth-2-yl]-trimethylammoniumchlorid, [8-((4-Amino-3-nitrophenyl)-azo)-7-hydroxy-naphth-2-yl]-trimethylammoniumchlorid (Basic Brown 17), 1-Hydroxy-2-amino-4,6-dinitrobenzol, 1-Amino-2-nitro-4-[bis-(2-hydroxyethyl)amino]-benzol, 1-Amino-2-[(2-hydroxyethyl)amino]-5-nitrobenzol (HC Yellow Nr.5), 1-Amino-2-nitro-4-[(2-hydroxyethyl)amino]-benzol (HC Red Nr. 7), 2-Chlor-5-nitro-N-2-hydroxyethyl-1,4-phenylendiamin, 1-[(2-Hydroxyethyl)amino]-2-nitro-4-amino-benzol (HC Red Nr. 3), 4-Amino-3-nitrophenol, 4-Amino-2-nitrophenol, 6-Nitro-o-toluidin, 1-Amino-3-methyl-4-[(2-hydroxyethyl)amino]-6-nitrobenzol (HC Violet Nr.1), 1-Amino-2-nitro-4-[(2,3-dihydroxypropyl)amino]-5-chlor-benzol (HC Red Nr. 10), 2-(4-Amino-2-nitroanilino)-benzoesäure, 6-Nitro-2,5-diaminopyridin, 2-Amino-6-chlor-4-nitrophenol, 1-Amino-2-(3-nitrophenylazo)-7-phenylazo-8-naphthol-3,6-disulfonsäure Dinatriumsalz (Acid blue Nr. 29), 1-Amino-2-(2-hydroxy-4-nitrophenylazo)-8-naphthol-3,6-disulfonsäure Dinatriumsalz (Palatinchrome green), 1-Amino-2-(3-chlor-2-hydroxy-5-nitrophenylazo)-8-naphthol-3,6-disulfonsäure Dinatriumsalz (Gallion), 4-Amino-4'-nitrostilben-2,2'-disulfonsäure Dinatriumsalz, 2,4-Diamino-3',5'-dinitro-2'-hydroxy-5-methyl-azobenzol (Mordant brown 4), 4'-Amino-4-nitrodiphenylamin-2-sulfonsäure, 4'-Amino-3'-nitrobenzophenon-2-carbonsäure, 1-Amino-4-nitro-2-(2-nitrobenzylidenamino)-benzol, 2-[2-(Diethylamino)ethylamino]-5-nitroanilin, 3-Amino-4-hydroxy-5-nitrobenzolsulfonsäure, 3-Amino-3'-nitrobiphenyl, 3-Amino-4-nitro-acenaphthen, 2-Amino-1-nitronaphthalin, 5-Amino-6-nitrobenzo-1,3-dioxol, Aniline, insbesondere Nitrogruppen-haltige Aniline, wie 4-Nitroanilin, 2-Nitroanilin, 1,4-Diamino-2-nitrobenzol, 1,2-Diamino-4-nitrobenzol, 1-Amino-2-methyl-6-nitrobenzol, 4-Nitro-1,3-phenylendiamin, 2-Nitro-4-amino-1-(2-hydroxyethylamino)-benzol, 2-Nitro-1-amino-4-[bis-(2-hydroxyethyl)-amino]-benzol, 4-Amino-2-nitrodiphenylamin-2'-carbonsäure, 1-Amino-5-chlor-4-(2-hydroyethylamino)-2-nitrobenzol, aromatische Aniline bzw. Phenole mit einem weiteren aromatischen Rest, wie sie in der Formel III dargestellt sind 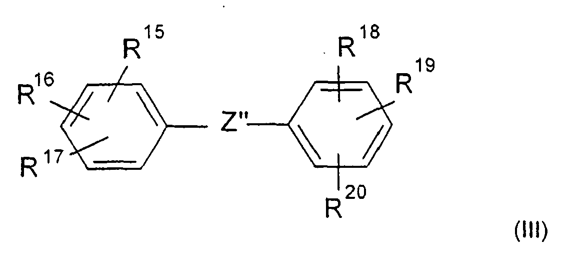 in der
- R¹⁵ für eine Hydroxy- oder eine Aminogruppe, die durch C₁-C₆-Alkyl-, C₁-C₆-Hydroxyalkyl-, C₁-C₆-Alkoxy- oder C₁-C₆-Alkoxy-C₁-C₆-alkylgruppen substituiert sein kann, steht,
- R¹⁶, R¹⁷, R¹⁸, R¹⁹ und R²⁰ unabhängig voneinander für ein Wasserstoffatom, eine Hydroxy- oder eine Aminogruppe, die durch C₁-C₆-Alkyl-, C₁-C₆-Hydroxyalkyl-, C₁-C₆-Alkoxy-, C₁-C₆-Aminoalkyl- oder C₁-C₆-Alkoxy-C₁-C₆-alkylgruppen substituiert sein kann, stehen, und
- Z" für eine direkte Bindung, eine gesättigte oder ungesättigte, ggf. durch Hydroxygruppen substituierte Kohlenstoffkette mit 1 bis 4 Kohlenstoffatomen, eine Carbonyl-, Sulfonyl- oder Iminogruppe, ein Sauerstoff- oder Schwefelatom, oder eine Gruppe mit der Formel IV 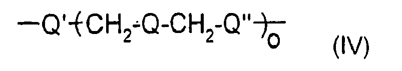 in der
- Q eine direkte Bindung, eine CH₂- oder CHOH-Gruppe bedeutet,
- Q' und Q" unabhängig voneinander für ein Sauerstoffatom, eine NR²¹-Gruppe, worin R²¹ ein Wasserstoffatom, eine C₁-C₆-Alkylgruppe oder C₁-C₆-Hydroxyalkylgruppe, wobei auch beide Gruppen zusammen mit dem Restmolekül einen 5-, 6- oder 7-Ring bilden können, bedeutet, die Gruppe O-(CH₂)ₚ-NH oder NH-(CH₂)ₚ'-O, worin p und p' 2 oder 3 sind, stehen und
- o eine Zahl von 1 bis 4 bedeutet,
wie insbesondere 4,4'-Diaminostilben und dessen Hydrochlorid, 4,4'-Diaminostilben-2,2'-disulfonsäure-mono- oder -di-Na-Salz, 4-Amino-4'-dimethylaminostilben und dessen Hydrochlorid, 4,4'-Diaminodiphenylmethan, 4,4'-Diaminodiphenylsulfid, 4,4'-Diaminodiphenylsulfoxid, 4,4'-Diaminodiphenylamin, 4,4'-Diaminodiphenylamin-2-sulfonsäure, 4,4'-Diaminobenzophenon, 4,4'-Diaminodiphenylether, 3,3',4,4'-Tetraaminodiphenyl, 3,3',4,4'-Tetraamino-benzophenon, 1,3-Bis-(2,4-diaminophenoxy)-propan, 1,8-Bis-(2,5-diaminophenoxy)-3,6-dioxaoctan, 1,3-Bis-(4-aminophenylamino)propan, , 1,3-Bis-(4-aminophenylamino)-2-propanol, 1,3-Bis-[N-(4-aminophenyl)-2-hydroxyethylamino]-2-propanol, N,N-Bis-[2-(4-aminophenoxy)-ethyl]-methylamin, N-Phenyl-1,4-phenylendiamin und Bis-(5-amino-2-hydroxyphenyl)-methan.

Die stickstoffhaltigen heterozyklischen Verbindungen sind bevorzugt ausgewählt aus der Gruppe bestehend aus 2-Aminopyridin, 3-Aminopyridin, 4-Aminopyridin, 2-Amino-3-hydroxy-pyridin, 2,6-Diamino-pyridin, 2,5-Diamino-pyridin, 2-(Aminoethylamino)-5-aminopyridin, 2,3-Diamino-pyridin, 2-Dimethylamino-5-amino-pyridin, 2-Methylamino-3-amino-6-methoxy-pyridin, 2,3-Diamino-6-methoxy-pyridin, 2,6-Dimethoxy-3,5-diamino-pyridin, 2,4,5-Triamino-pyridin, 2,6-Dihydroxy-3,4-dimethylpyridin, N-[2-(2,4-Diaminophenyl)aminoethyl]-N-(5-amino-2-pyridyl)-amin, N-[2-(4-Aminophenyl)aminoethyl]-N-(5-amino-2-pyridyl)-amin, 2,4-Dihydroxy-5,6-diaminopyrimidin, 4,5,6-Triaminopyrimidin, 4-Hydroxy-2,5,6-triaminopyrimidin, 2-Hydroxy-4,5,6-triaminopyrimidin, 2,4,5,6-Tetraaminopyrimidin, 2-Methylamino-4,5,6-triaminopyrimidin, 2,4-Diaminopyrimidin, 4,5-Diaminopyrimidin, 2-Amino-4-methoxy-6-methyl-pyrimidin, 3,5-Diaminopyrazol, 3,5-Diamino-1,2,4-triazol, 3-Aminopyrazol, 3-Amino-5-hydroxypyrazol, 1-Phenyl-4,5-diaminopyrazol, 1-(2-Hydroxyethyl)-4,5-diaminopyrazol, 1-Phenyl-3-methyl-4,5-diaminopyrazol, 4-Amino-2,3-dimethyl-1-phenyl-3-pyrazolin-5-on (4-Aminoantipyrin), 1-Phenyl-3-methylpyrazol-5-on, 2-Aminochinolin, 3-Aminochinolin, 8-Aminochinolin, 4-Aminochinaldin, 2-Aminonicotinsäure, 6-Aminonicotinsäure, 5-Aminoisochinolin, 5-Aminoindazol, 6-Aminoindazol, 5-Aminobenzimidazol, 7-Aminobenzimidazol, 5-Aminobenzothiazol, 7-Aminobenzothiazol, 2,5-Dihydroxy-4-morpholino-anilin sowie Indol- und Indolinderivaten, wie 4-Aminoindol, 5-Aminoindol, 6-Aminoindol, 7-Aminoindol, 5,6-Dihydroxyindol, 5,6-Dihydroxyindolin und 4-Hydroxyindolin. Weiterhin als heterozyklische Verbindungen können erfindungsgemäß die in der DE-U1-299 08 573 offenbarten Hydroxypyrimidine eingesetzt werden. Die vorgenannten Verbindungen können sowohl in freier Form als auch in Form ihrer physiologisch verträglichen Salze, z. B. als Salze anorganischer Säuren, wie Salz- oder Schwefelsäure, eingesetzt werden.

Die voranstehend genannten zusätzlichen farbgebenden Komponenten können in einer Menge von jeweils 0,03 bis 65 mmol, insbesondere 1 bis 40 mmol, jeweils bezogen auf 100 g der gesamten Anwendungsmischung, eingesetzt werden. Weiterhin kann es erfindungsgemäß besonders bevorzugt sein, die Verbindung der Formel 1 und die Verbindung der zusätzlichen farbgebenden Komponente im molaren Mengenverhältnis von 2 : 1 bis 1 : 2, insbesondere etwa äquimolar, einzusetzen. Beim Einsatz von Verbindungen der Komponente B, die eine oder mehrere Aminogruppen enthalten, werden die Verbindungen der Formel I einerseits und die zusätzlichen farbgebenden Verbindungen andererseits bevorzugt in solchen Mengenverhältnissen eingesetzt, daß die Summe der Zahl der Carbonylgruppen der Verbindungen der Formel I und die Summe der Zahl der Aminogruppen der Verbindungen der zusätzlichen farbgebenden Verbindungen im Verhältnis 2 : 1 bis 1 : 2 stehen.

Zur Erlangung weiterer und intensiverer Ausfärbungen können die erfindungsgemäßen Zusammensetzungen können zusätzlich Farbverstärker enthalten. Die Farbverstärker sind vorzugsweise ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Histidin, Pyrrolidin, Prolin, Pyrrolidon, Pyrrolidon-5-carbonsäure, Pyrazol, 1,2,4-Triazol, Piperazidin, deren Derivate sowie deren physiologisch verträglichen Salzen.

Die voranstehend genannten Farbverstärker können in einer Menge von jeweils 0,03 bis 65 mmol, insbesondere 1 bis 40 mmol, jeweils bezogen auf 100 g des gesamten Färbemittels, eingesetzt werden.

Für das Färbeergebnis kann es vorteilhaft sein, den Färbemitteln Ammonium- oder Metallsalze zuzugeben. Geeignete Metallsalze sind z.B. Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Erdalkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, wobei Natriumacetat, Lithiumbromid, Calciumbromid, Calciumgluconat, Zinkchlorid, Zinksulfat, Magnesiumchlorid, Magnesiumsulfat, Ammoniumcarbonat, -chlorid und -acetat bevorzugt sind. Diese Salze sind vorzugsweise in einer Menge von 0,03 bis 65, insbesondere von 1 bis 40 mmol, bezogen auf 100 g des gesamten Färbemittels, enthalten.

Auf die Anwesenheit von zusätzlichen chemischen Oxidationsmitteln, z.B. H₂O₂, wird bevorzugt in den erfindungsgemäßen Mitteln verzichtet. Es kann jedoch u.U. wünschenswert sein, den erfindungsgemäßen Zusammensetzungen zur Erzielung von Nuancen, die heller als die zu färbende keratinhaltige Faser sind, Wasserstoffperoxid oder andere Oxidationsmittel zuzusetzen. Oxidationsmittel werden in der Regel in einer Menge von 0,01 bis 6 Gew.-%, bezogen auf die Anwendungslösung, eingesetzt. Ein für menschliches Haar bevorzugtes Oxidationsmittel ist H₂O₂.

In einer weiteren Ausführungsform enthalten die erfindungsgemäßen Zusammensetzungen zur weiteren Modifizierung der Farbnuancen neben den erfindungsgemäß enthaltenen Verbindungen zusätzlich übliche direktziehende Farbstoffe. Die direktziehenden Farbstoffe können z. B. ausgewählt sein aus der Gruppe der Nitrophenylendiamine, Nitroaminophenole, Anthrachinone oder Indophenole, wie z.B. die unter den internationalen Bezeichnungen bzw. Handelsnamen HC Yellow 2, HC Yellow 4, HC Yellow 6, Basic Yellow 57, Disperse Orange 3, HC Red 3, HC Red BN, Basic Red 76, HC Blue 2, Disperse Blue 3, Basic Blue 99, HC Violet 1, Disperse Violet 1, Disperse Violet 4, Disperse Black 9, Basic Brown 16, Basic Brown 17, Pikraminsäure und 2-Amino-6-chloro-4-nitrophenol bekannten Verbindungen sowie 4-Amino-2-nitrodiphenylamin-2'-carbonsäure, 6-Nitro-1,2,3,4-tetrahydrochinoxalin, 4-N-Ethyl-1,4-bis(2'-hydroxyethylamino)-2-nitrobenzol-hydrochlorid und 1-Methyl-3-nitro-4-(2'-hydroxyethyl)-aminobenzol. Die erfindungsgemäßen Mittel gemäß dieser Ausführungsform enthalten die direktziehenden Farbstoffe bevorzugt in einer Menge von 0,01 bis 20 Gew.-%, bezogen auf die gesamte Anwendungsmischung.

Die aus den erfindungsgemäßen Zusammensetzungen herstellbaren Färbemittel ergeben bereits bei physiologisch verträglichen Temperaturen von unter 45°C intensive Färbungen. Sie eignen sich deshalb besonders zum Färben von menschlichen Haaren. Zur Anwendung auf dem menschlichen Haar können die erfindungsgemäße Zusammensetzungen üblicherweise in einen wasserhaltigen kosmetischen Träger eingearbeitet werden. Geeignete wasserhaltige kosmetische Träger sind z.B. Cremes, Emulsionen, Gele oder auch tensidhaltige schäumende Lösungen wie z.B. Shampoos oder andere Zubereitungen, die für die Anwendung auf den keratinhaltigen Fasern geeignet sind.

Weiterhin können die erfindungsgemäßen Zusammensetzungen alle in solchen Zubereitungen bekannten Wirk-, Zusatz- und Hilfsstoffe enthalten. In vielen Fällen enthalten die Zusammensetzungen mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethyl-ammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Zusammensetzungen verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid^{®}S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex^{®} vertriebenen Methylhydroxyalkyldialkoyloxyalkylammoniummethosulfate.

Ein Beispiel für ein als kationisches Tensid einsetzbares quaternäres Zuckerderivat stellt das Handelsprodukt Glucquat^{®} 100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Die Tenside werden bevorzugt in Konzentrationen von 0,5 bis 30 Gew.-%, bezogen auf das Gewicht der Zusammensetzung, eingesetzt.

Die erfindungsgemäßen Zusammensetzungen können zusätzlich einen Fettalkohol enthalten. Unter Fettalkoholen sind primäre aliphatische Monoalkohole mit einem aliphatischen, linearen oder verzweigten Kohlenwasserstoffrest mit 6 bis 30 Kohlenstoffatomen und 0 und/oder 1, 2 oder 3 Doppelbindungen steht. Typische Beispiele sind Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalko-hol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Bevorzugt sind technische Fettalkohole mit 12 bis 18 Kohlenstoffatomen, wie beispielsweise Kokos-, Palm-, Palmkern- oder Talgfettalkohol.

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von linearen und/oder verzweigten Fettsäuren mit mehrwertigen Alkoholen (wie z.B. Propylenglycol, Dimer-diol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, pflanzliche Öle, verzweigte primäre Alkohole, substituierte Cyclohexane, lineare C₆-C₂₂-Fettalkoholcarbonate, Guerbetcarbonate, Ester der Benzoesäure mit linearen und/oder verzweigten C₆-C₂₂-Alkoholen (z.B. Finsolv^{®} TN), Dialkylether, Ringöffnungsprodukte von epoxidierten Fettsäureestern mit Polyolen, Siliconöle und/oder aliphatische bzw. naphthenische Kohlenwasserstoffe in Betracht.

Weitere Wirk-, Hilfs- und Zusatzstoffe sind beispielsweise
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konservierungsmittel, wie z.B. Parabene und Phenoxyethanol,
- Fettsäurealkanolamide,
- Komplexbildner wie EDTA, NTA und Phosphonsäuren,
- Quell- und Penetrationsstoffe wie Propylenglykolmonoethylether, Carbonate, Hydrogencarbonate, Guanidine, Harnstoffe sowie primäre, sekundäre und tertiäre Phosphate, Imidazole, Tannine, Pyrrol,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Ein dritter Gegenstand der Erfindung ist ein Verfahren zum Färben keratinhaltiger Fasern, insbesondere menschlicher Haare, in welchem
(i) unmittelbar vor der Anwendung eine erfindungsgemäße Zusammensetzung des zweiten Gegenstandes der Erfindung mit einer zweiten Zusammensetzung, enthaltend mindestens eine CH-acide Verbindung und/oder mindestens eine Verbindung mit primärer oder sekundärer Aminogruppe, ausgewählt aus der Gruppe, die gebildet wird aus primären oder sekundären aromatischen Aminen, stickstoffhaltigen heterocyclischen Verbindungen und aromatischen Hydroxyverbindungen, gemischt wird,
(ii) die resultierende Anwendungsmischung auf die Faser appliziert und
(iii) nach einer Einwirkzeit wieder abgespült wird.

Die erfindungsgemäßen primären oder sekundären Amine, bzw. die CH-aciden Verbindungen wurden zuvor definiert. Es ist in dem erfindungsgemäßen Verfahren bevorzugt, dass die erfindungsgemäße Zusammensetzung frei ist von CH-aciden Verbindungen und Verbindungen mit primärer oder sekundärer Aminogruppe, ausgewählt aus der Gruppe, die gebildet wird aus primären oder sekundären aromatischen Aminen, stickstoffhaltigen heterocyclischen Verbindungen und aromatischen Hydroxyverbindungen.

Zum Färben der keratinhaltigen Fasern, insbesondere zum Färben von menschlichen Haaren, werden die erfindungsgemäßen Mittel in der Regel gemeinsam mit einem wasserhaltigen, kosmetischen Träger als Anwendungsmischung des Schritts (ii) in einer Menge von 100 g auf das Haar aufgebracht, ca. 30 Minuten dort belassen und anschließend ausgespült oder mit einem handelsüblichen Haarshampoo ausgewaschen. Die fakultativ enthaltenen Ammonium- oder Metallsalze können allein oder im Gemisch mit den weiteren Komponenten zugesetzt werden. Auch eine Vorbehandlung der Fasern mit der Salzlösung ist möglich.

Der pH-Wert der gebrauchsfertigen Anwendungsmischungen liegt üblicherweise zwischen 2 und 11, vorzugsweise zwischen 6 und 10. Es ist bevorzugt, dass die zweite Zusammensetzung gemäß Schritt (i) des Verfahrens einen pH von 2 bis 11 besitzt und die erfindungsgemäß beanspruchte Zusammensetzung auf einen pH-Wert von 1 bis 7, bevorzugt von 3 bis 6, eingestellt ist.

Das Mischverhalten der beiden Zusammensetzungen aus Schritt (i) des erfindungsgemäßen Verfahrens ist optimal, wenn die zweite Zusammensetzung gemäß Schritt (i) des Verfahrens eine Viskosität von 15000 bis 150000 mPa·s besitzt und die erfindungsgemäß beanspruchte Zusammensetzung auf eine Viskosität von 50 bis 100000 mPa·s hat. Die Anwendungsmischung erhält eine Viskosität von 5000 bis 50000 mPa·s, lässt sich leicht auftragen und erlaubt eine rasche Penetration der farbstoffbildenden Komponenten in das Haar. Alle Viskositäten werden bei 20°C mit einem Rotationsviskosimeter mit Spindel Nr. 4 bestimmt.

Zur getrennten Lagerung der in Schritt (i) des erfindungsgemäßen Verfahrens verwendeten Zusammensetzungen werden die Zusammensetzungen dem Verbraucher in einem Kit-of-parts angeboten, in welchem die jeweiligen Zusammensetzungen räumlich getrennt aufbewahrt werden. Dieses Kit-of-parts kann aus einem einzigen Behältnis z.B. in Form einer Flasche oder Tube bestehen, welches jeweils eine Kammer zur getrennten Lagerung der Zusammensetzungen aus Schritt (i) des erfindungsgemäßen Verfahrens besitzt. Die Anwendungsmischung kann dann entweder durch in Kontakt bringen der Zusammensetzungen innerhalb dieser Einheit, oder durch in Kontakt bringen während des Austritts aus der Einheit bewerkstelligt werden.

Eine weitere Möglichkeit bietet die getrennte Konfektionierung der Zusammensetzungen in jeweils einem eigenständigen Behältnis.

### Beispiele

Es wurden folgende gelförmige Zusammensetzungen gemäß Tabelle 1 durch Mischen der entsprechenden Komponenten hergestellt. Die Lagerstabilität jeder einzelnen Mischung wurde in einem verschlossenen Gefäß über einen Zeitraum von sechs Monaten bei Raumtemperatur bestimmt. Täglich wurde die Stabilität überprüft.

Die in Tabelle 1 verzeichneten Mengenangaben der Rohstoffe sind Gewichtsprozent, bezogen auf das Gewicht der fertigen Mischung.

Es ist festzustellen, dass die erfindungsgemäßen Zusammensetzungen A, B und C eine wesentlich längere Lagerstabilität bei Raumtemperatur besitzen, als die nicht erfindungsgemäße Rezeptur D. Bereits vor Ablauf eines Monats war eine Kristallbildung in Rezeptur D eingetreten.

**Tabelle 1:Rezepturen und Ergebnisse des Stabilitätstests**

| Komponenten | **A** | **B** | **C** | **D** |
|---|---|---|---|---|
| 4-Formyl-1-methylchinolinium-p-toluolsulfonat | 2,75 | 2,75 | 2,75 | 2,75 |
| Ethylenglykol | 15,00 | - | - | - |
| Diethylenglykol | - | 18,00 | - | - |
| 1,2-Propylenglykol | - | - | 5,00 | - |
| Konservierungsmittel | 1,20 | 1,20 | 1,20 | 1,20 |
| Natrosol^{®} 25OHR ¹ | 1,00 | 1,00 | 1,00 | 1,00 |
| Parfum | 0,10 | 0,10 | 0,10 | 0,10 |
| Wasser, dest. | ad 100 | ad 100 | ad 100 | ad 100 |
| Lagerstabilität bei Raumtemperatur | > 6 Monate > | 6 Monate < | 3 Monate < | 1 Monat |

| | | | | |
|---|---|---|---|---|
| Hydroxyethylcellulose (Hercules) | | | | |

## Patentansprüche

1. Zusammensetzung, enthaltend eine Kombination aus Komponente
(i) mindestens eines Oniumaldehyds und -ketons der folgenden Formel l oder deren Derivaten 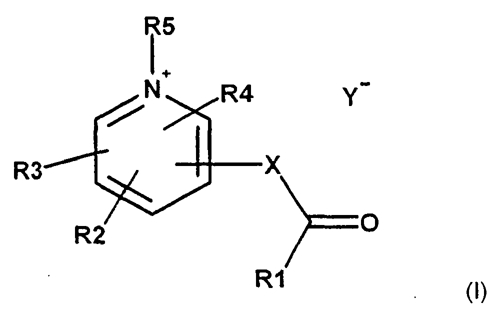 worin
R1 für ein Wasserstoffatom, eine (C₁-C₆)-Alkylgruppe, eine Arylgruppe oder eine Heteroarylgruppe steht,
R2, R3 und R4 jeweils unabhängig voneinander ein Wasserstoffatom, ein Halogenatom, eine (C₁-C₆)-Alkylgruppe, (C₁-C₆)-Alkoxygruppe, (C₁-C₆)-Hydroxylalkylgruppe, Hydroxy-(C₁-C₆)-alkoxygruppe, Hydroxygruppe, Nitrogruppe, Arylgruppe, Trifluormethylgruppe, Aminogruppe, die durch (C₁-C₆)-Alkylgruppen substituiert sein kann, oder (C₁-C₆)-Acylgruppe bedeuten, wobei auch zwei der Reste gemeinsam einen ankondensierten Benzolring bilden können,
R5 für eine (C₁-C₆)-Alkylgruppe, Arylgruppe, Aryl-(C₁-C₆)-alkygruppe oder Heteroarylgruppe steht,
X für eine direkte Bindung oder eine ggf. substituierte Vinylen- oder Phenylengruppe steht, und
Y⁻ Halogenid, Benzolsulfonat, p-Toluolsulfonat, Methansulfonat, Trifluormethansulfonat, Perchlorat, Sulfat, Hydrogensulfat, Tetrachlorzinkat oder N-Oxid des Heterocyclus bedeutet,
mit Komponente
(ii) 2 bis 50 Gew.-%, bezogen auf das Gewicht der gesamten Zusammensetzung, mindestens eines linearen oder verzweigten, aliphatischen Polyols und
(iii) Wasser.

2. Zusammensetzung nach Anspruch 1, **dadurch gekennzeichnet, dass** die Verbindungen gemäß Formel (I) ausgewählt sind aus Verbindungen gemäß Formel (ll) 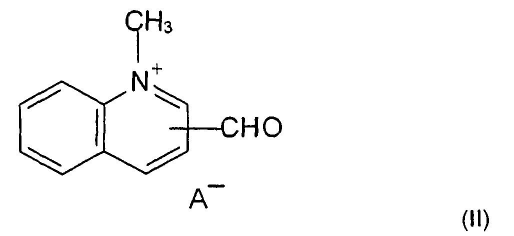 worin die Formylgruppe CHO in 2- oder 4-Position gebunden ist und A⁻ steht für ein p-Toluolsulfonat-, Methansulfonat-, Trifluormethansulfonat-, Methylsulfat- oder Benzolsulfonat-lon.

3. Zusammensetzung nach Anspruch 1, **dadurch gekennzeichnet, daß** die Komponente (i) ausgewählt ist der Gruppe bestehend aus den Benzolsulfonaten, p-Toluolsulfonaten, Methansulfonaten, Trifluormethansulfonaten, Perchloraten, Sulfaten, Chloriden, Bromiden, Jodiden und/oder Tetrachlorzinkaten von 4-Formyl-1-methylpyridinium, 3-Formyl-1-methylpyridinium, 2-Formyl-1-methylpyridinium, 4-Formyl-1-ethylpyridinium, 2-Formyl-1-ethylpyridinium, 4-Formyl-1-benzylpyridinium, 2-Formyl-1-benzylpyridinium, 4-Formyl-1,2-dimethyl-pyridinium, 4-Formyl-1,3-dimethylpyridinium, 4-Formyl-1-methylchinolinium, 2-Formyl-1-methylchinolinium, 4-(2-Formylvinyl)-1-methylchinolinium, 4-Acetyl-1-methylpyridinium, 2-Acetyl-1-methylpyridinium, 4-Acetyl-1-chinolinium, 4-Acetyl-1-methylchinolinium und 4-(2-Formylvinyl)-1-methylpyridinium, 2,6-Dichlor-4-formyl-1-methylpryridinium, 2,6-Diphenyl-4-formyl-1-methylpyridinium, 4-Benzoyl-1-methylpyridinium, 4-Propionyl-1-methylpyridinium, 2-Oximomethyl-1-methylpyridinium, 4-Pyridincarboxaldehyd-N-oxid und N-Methylpyridoxal.

4. Zusammensetzung nach einem der Ansprüche 1 bis 3, **dadurch gekennzeichnet, daß** die Komponente (A) als Hydrat vorliegt.

5. Zusammensetzung, nach einem der Ansprüche 1 bis 4, **dadurch gekennzeichnet, daß** die linearen oder verzweigten, aliphatischen Polyole ausgewählt werden aus einer Gruppe, die gebildet wird aus HO-(CH₂CH₂O)ₙ-H mit n = 1 bis 14, Dipropylenglykol, Tripropylenglykol, Glycerin, Diglycerin, 1,2-Butandiol, 1,4-Butandiol, 1,5-Pentandiol, 1,2-Pentandiol, 2-Methyl-2,4-pentandiol, 4-Methyl-2-pentanol, 1,2,6-Hexantriol und 2-Ethyl-1,3-hexandiol.

6. Zusammensetzung nach einem der Ansprüche 1 bis 5, **dadurch gekennzeichnet, daß** sie zusätzlich mindestens ein Polymer, ausgewählt aus anionischen, kationischen, nichtionischen und amphoteren Polymeren, enthält.

7. Zusammensetzung nach Anspruch 6, **dadurch gekennzeichnet, daß** das Polymer ein nichtionisches und/oder kationisches Polymer ist.

8. Zusammensetzung nach einem der Ansprüche 6 oder 7, **dadurch gekennzeichnet, daß** das Polymer in einer Menge von 0.05 bis 5 Gew.-% bezogen auf das Gewicht der Zusammensetzung enthalten ist.

9. Zusammensetzung nach einem der Ansprüche 1 bis 8, **dadurch gekennzeichnet, daß** sie zusätzlich mindestens ein Tensid, ausgewählt aus anionischen, kationischen, nichtionischen, amphoteren und zwitterionischen Tensiden, enthält

10. Zusammensetzung nach einem der Ansprüche 1 bis 9, **dadurch gekennzeichnet, daß** sie zusätzlich mindestens einen Fettalkohol mit 6 bis 30 Kohlenstoffatomen enthält.

11. Zusammensetzung nach einem der Ansprüche 1 bis 10, **dadurch gekennzeichnet, daß** sie als Creme, Emulsion, Gel oder schäumende Lösung vorliegt.

12. Verwendung von 2 bis 50 Gew.%, bezogen auf das Gewicht der wässrigen Zusammensetzung, eines aliphatischen, linearen oder verzweigten Polyols zur Kristallisationsinhibierung von Oniumaldehyden und -ketonen oder deren Derivaten gemäß einem der Ansprüche 1 bis 4 in wässrigen Zusammensetzungen.

13. Verfahren zum Färben von keratinhaltigen Fasern, insbesondere menschlichen Haaren, **dadurch gekennzeichnet, daß**
(i) unmittelbar vor der Anwendung eine Zusammensetzung nach einem der Ansprüche 1 bis 11 mit einer zweiten Zusammensetzung, enthaltend mindestens eine CH-acide Verbindung und/oder mindestens eine Verbindung mit primärer oder sekundärer Aminogruppe, ausgewählt aus der Gruppe, die gebildet wird aus primären oder sekundären aromatischen Aminen, stickstoffhaltigen heterocyclischen Verbindungen und aromatischen Hydroxyverbindungen, gemischt wird,
(ii) die resultierende Anwendungsmischung auf die Faser appliziert und
(iii) nach einer Einwirkzeit wieder abgespült wird.

14. Kit-of-parts enthaltend eine Zusammensetzung nach einem der Ansprüche 1 bis 11 und eine Zusammensetzung, enthaltend mindestens eine CH-acide Verbindung und/oder mindestens eine Verbindung mit primärer oder sekundärer Aminogruppe, ausgewählt aus der Gruppe, die gebildet wird aus primären oder sekundären aromatischen Aminen, stickstoffhaltigen heterocyclischen Verbindungen und aromatischen Hydroxyverbindungen, wobei die Zusammensetzungen in einem Behältnis mit getrennten Kammern, oder jeweils in einem eigenständigen Behältnis, getrennt voneinander konfektioniert sind.

## Claims

1. Composition comprising a combination of component
(i) at least one onium aldehyde and onium ketone of the following formula I or derivatives thereof 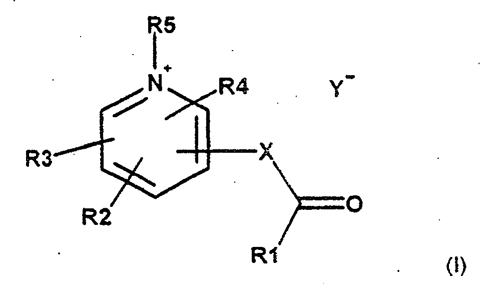 in which
R1 is a hydrogen atom, a (C₁-C₆)-alkyl group, an aryl group or a heteroaryl group,
R2, R3 and R4, in each case independently of one another, are a hydrogen atom, a halogen atom, a (C₁-C₆) -alkyl group, (C₁-C₆) -alkoxy group, (C₁-C₆) - hydroxyalkyl group, hydroxy-(C₁-C₆)-alkoxy group, hydroxy group, nitro group, aryl group, trifluoromethyl group, amino group, which may be substituted by (C₁-C₆)-alkyl groups, or (C₁-C₆)-acyl group, where also two of the radicals can together form a fused-on benzene ring,
R5 is a (C₁-C₆)-alkyl group, aryl group, aryl-(C₁-C₆)-alkyl group or heteroaryl group,
X is a direct bond or an optionally substituted vinylene or phenylene group, and
Y⁻ is halide, benzenesulphonate, p-toluenesulphonate, methanesulphonate, trifluoromethanesulphonate, perchlorate, sulphate, hydrogensulphate, tetrachlorozincate or N-oxide of the heterocycle,
with component
(ii) 2 to 50% by weight, based on the weight of the total composition, of at least one linear or branched, aliphatic polyol and
(iii) water.

2. Composition according to Claim 1, **characterized in that** the compounds according to formula (I) are selected from compounds according to formula (II) 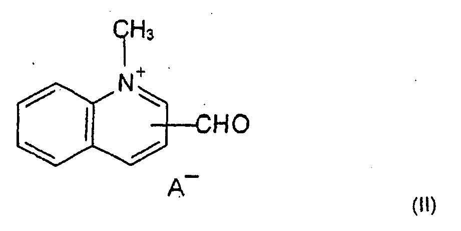 in which the formyl group CHO is bonded in the 2- or 4-position and A⁻ is a p-toluenesulphonate, methanesulphonate, trifluoromethanesulphonate, methylsulphate or benzenesulphonate ion.

3. Composition according to Claim 1, **characterized in that** component (i) is selected from the group consisting of the benzenesulphonates, p-toluene-sulphonates, methanesulphonates, trifluoromethane-sulphonates, perchlorates, sulphates, chlorides, bromides, iodides and/or tetrachlorozincates of 4-formyl-1-methylpyridinium, 3-formyl-1-methyl-pyridinium, 2-formyl-1-methylpyridinium, 4-formyl-1-ethylpyridinium, 2-formyl-1-ethylpyridinium, 4-formyl-1-benzylpyridinium, 2-formyl-1-benzyl-pyridinium, 4-formyl-1,2-dimethylpyridinium, 4-formyl-1,3-dimethylpyridinium, 4-formyl-1-methylquinolinium, 2-formyl-1-methylquinolinium, 4-(2-formylvinyl)-1-methylquinolinium, 4-acetyl-1-methylpyridinium, 2-acetyl-1-methylpyridinium, 4-acetyl-1-quinolinium, 4-acetyl-1-methylquinolinium and 4-(2-formylvinyl)-1-methylpyridinium, 2,6-dichloro-4-formyl-1-methylpyridinium, 2,6-diphenyl-4-formyl-1-methylpyridinium, 4-benzoyl-1-methylpyridinium, 4-propionyl-1-methylpyridinium, 2-oximomethyl-1-methylpyridinium, 4-pyridine-carboxaldehyde N-oxide and N-methylpyridoxal.

4. Composition according to one of Claims 1 to 3, **characterized in that** component (A) is present as hydrate.

5. Composition according to one of Claims 1 to 4, **characterized in that** the linear or branched, aliphatic polyols are selected from the group which is formed from HO-(CH₂CH₂O)ₙ-H where n = 1 to 14, dipropylene glycol, tripropylene glycol, glycerol, diglycerol, 1,2-butanediol, 1,4-butanediol, 1,5-pentanediol, 1,2-pentanediol, 2-methyl-2,4-pentanediol, 4-methyl-2-pentanol, 1,2,6-hexanetriol and 2-ethyl-1,3-hexanediol.

6. Composition according to one of Claims 1 to 5, **characterized in that** it additionally comprises at least one polymer selected from anionic, cationic, nonionic and amphoteric polymers.

7. Composition according to Claim 6, **characterized in that** the polymer is a nonionic and/or cationic polymer.

8. Composition according to one of Claims 6 or 7, **characterized in that** the polymer is present in an amount of from 0.05 to 5% by weight, based on the weight of the composition.

9. Composition according to one of Claims 1 to 8, **characterized in that** it additionally comprises at least one surfactant selected from anionic, cationic, nonionic, amphoteric and zwitterionic surfactants.

10. Composition according to one of Claims 1 to 9, **characterized in that** it additionally comprises at least one fatty alcohol having 6 to 30 carbon atoms.

11. Composition according to one of Claims 1 to 10, **characterized in that** it is in the form of a cream, emulsion, gel or foaming solution.

12. Use of from 2 to 50% by weight, based on the weight of the aqueous composition, of an aliphatic, linear or branched polyol for inhibiting the crystallization of onium aldehydes and onium ketones or derivatives thereof according to one of Claims 1 to 4 in aqueous compositions.

13. Method of colouring keratin-containing fibres, in particular human hair, **characterized in that**
(i) immediately prior to application, a composition according to one of Claims 1 to 11 is mixed with a second composition, comprising at least one CH-acidic compound and/or at least one compound with a primary or secondary amino group selected from the group which is formed from primary or secondary aromatic amines, nitrogen-containing heterocyclic compounds and aromatic hydroxy compounds,
(ii) the resulting application mixture is applied to the fibre and
(iii) after a contact time, is rinsed out again.

14. Kit-of-parts comprising a composition according to one of Claims 1 to 11 and a composition comprising at least one CH-acidic compound and/or at least one compound with a primary or secondary amino group selected from the group which is formed from primary or secondary aromatic amines, nitrogen-containing heterocyclic compounds and aromatic hydroxy compounds, where the compositions have been formulated separately from one another in one container with separate chambers, or each in an independent container.

## Revendications

1. Composition contenant une combinaison du composant
(i) au moins un aldéhyde et une cétone à radical onium répondant à la formule I suivante ou leurs dérivés 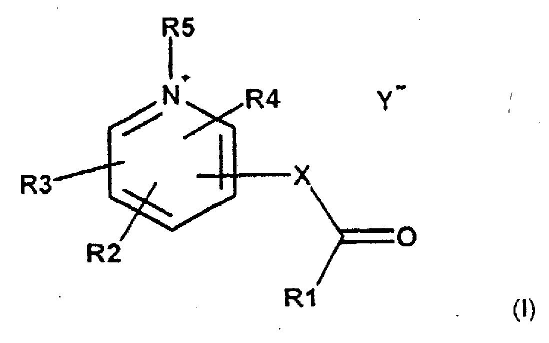 dans laquelle
R1 représente un atome d'hydrogène, un groupe alkyle en C₁-C₆, un groupe aryle ou un groupe hétéroaryle,
R2, R3 et R4 représentent, indépendamment l'un de l'autre, respectivement un atome d'hydrogène, un atome d'halogène, un groupe alkyle en C₁-C₆, un groupe alcoxy en C₁-C₆, un groupe hydroxyalkyle en C₁-C₆, un groupe hydroxyalcoxy en C₁-C₆, un groupe hydroxyle, un groupe nitro, un groupe aryle, un groupe trifluorométhyle, un groupe amino qui peut être substitué par des groupes alkyle en C₁-C₆, ou un groupe acyle en C₁-C₆, deux des radicaux pouvant également former ensemble un noyau benzénique condensé,
R5 représente un groupe alkyle en C₁-C₆, un groupe aryle, un groupe arylalkyle en C₁-C₆ ou un groupe hétéroaryle,
X représente une liaison directe ou bien un groupe vinylène ou un groupe phénylène éventuellement substitué, et
Y⁻ représente un halogénure, un benzènesulfonate, un p-toluènesulfonate, un méthanesulfonate, un trifluorométhanesulfonate, un perchlorate, un sulfate, un hydrogénosulfate, un tétrachlorozincate ou un N-oxyde du radical hétérocyclique,
avec un composant
(ii) à concurrence de 2 à 50 % en poids rapportés au poids de la composition totale, au moins un polyol aliphatique linéaire ou ramifié, et
(iii) de l'eau.

2. Composition selon la revendication 1, **caractérisée en ce que** les composés répondant à la formule (I) sont choisis parmi des composés répondant à la formule (II) 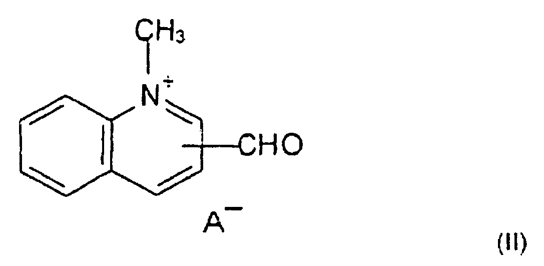 dans laquelle le groupe formyle CHO est relié en position 2 ou 4 et A⁻ représente un ion de p-toluènesulfonate, un ion de méthanesulfonate, un ion de trifluorométhanesulfonate, un ion de méthylsulfate ou un ion de benzènesulfonate.

3. Composition selon la revendication 1, **caractérisée en ce que** le composant (i) est choisi parmi le groupe constitué par les benzènesulfonates, les p-toluènesulfonates, les méthanesulfonates, les trifluorométhane-sulfonates, les perchlorates, les sulfates, les chlorures, les bromures, les iodures et/ou les tétrachlorozincates du 4-formyl-1-méthylpyridinium, du 3-formyl-1-méthylpyridinium, du 2-formyl-1-méthylpyridinium, du 4-formyl-1-éthylpyridinium, du 2-formyl-1-éthylpyridinium, du 4-formyl-1-benzylpyridinium, du 2-formyl-1-benzylpyridinium, du 4-formyl-1,2-diméthylpyridinium, du 4-formyl-1,3-diméthylpyridinium, du 4-formyl-1-méthylquinolinium, du 2-formyl-1-méthylquinolinium, du 4-(2-formylvinyl)-1-méthyl-quinolinium, du 4-acétyl-1-méthylpyridinium, du 2-acétyl-1-méthylpyridinium, du 4-acétyl-1-quinolinium, du 4-acétyl-1-méthyl-quinolinium et du 4-(2-formylvinyl)-1-méthylpyridinium, du 2,6-dichloro-4-formyl-1-méthylpyridinium, du 2,6-diphényl-4-formyl-1-méthylpyridinium, du 4-benzoyl-1-méthylpyridinium, du 4-propionyl-1-méthylpyridinium, du 2-oximométhyl-1-méthylpyridinium, du N-oxyde du 4-pyridinecarboxaldéhyde et du N-méthylpyridoxal.

4. Composition selon l'une quelconque des revendications 1 à 3, **caractérisée en ce que** le composant (A) est présent sous la forme d'un hydrate.

5. Composition selon l'une quelconque des revendications 1 à 4, **caractérisée en ce que** les polyols aliphatiques linéaires ou ramifiés sont choisis parmi un groupe qui est formé par un groupe HO-(CH₂-CH₂O)ₙ-H dans lequel n = 1 à 14, le dipropylèneglycol, le tripropylèneglycol, le glycérol, le diglycérol, le 1,2-butanediol, le 1,4-butanediol, le 1,5-pentanediol, le 1,2-pentanediol, le 2-méthyl-2,4-pentanediol, le 4-méthyl-2-pentanol, le 1,2,6-hexanetriol et le 2-éthyl-1,3-hexanediol.

6. Composition selon l'une quelconque des revendications 1 à 5, **caractérisée en ce qu'**elle contient en outre au moins un polymère choisi parmi des polymères anioniques, cationiques, non ioniques et amphotères.

7. Composition selon la revendication 6, **caractérisée en ce que** le polymère est un polymère non ionique et/ou cationique.

8. Composition selon l'une quelconque des revendications 6 ou 7, **caractérisée en ce que** le polymère est présent en une quantité de 0,05 à 5 % en poids, rapportés au poids de la composition.

9. Composition selon l'une quelconque des revendications 1 à 8, **caractérisée en ce qu'**elle contient en outre au moins un agent tensioactif choisi parmi des agents tensioactifs anioniques, cationiques, non ioniques, amphotères et zwitterioniques.

10. Composition selon l'une quelconque des revendications 1 à 9, **caractérisée en ce qu'**elle contient en outre au moins un alcool gras contenant de 6 à 30 atomes de carbone.

11. Composition selon l'une quelconque des revendications 1 à 10, **caractérisée en ce qu'**elle est présente sous la forme d'une crème, d'une émulsion, d'un gel ou d'une solution moussante.

12. Utilisation, à concurrence de 2 à 50 % en poids, rapportés au poids de la composition aqueuse, d'un polyol aliphatique linéaire ou ramifié pour inhiber la cristallisation d'aldéhydes et de cétones à radicaux onium ou de leurs dérivés selon l'une quelconque des revendications 1 à 4 dans des compositions aqueuses.

13. Procédé pour la teinture de fibres kératiniques, en particulier de cheveux humains, **caractérisé en ce que**
(i) directement avant l'application, on mélange une composition selon l'une quelconque des revendications 1 à 11 avec une deuxième composition contenant au moins un composé à CH acide et/ou au moins un composé contenant un groupe amino primaire ou secondaire choisi parmi le groupe qui est formé par des amines aromatiques primaires ou secondaires, des composés hétérocycliques azotés et des composés hydroxylés aromatiques,
(ii) on applique sur les fibres le mélange d'utilisation résultant, et
(iii) après avoir laissé agir pendant un certain temps, on l'élimine par rinçage.

14. Nécessaire en plusieurs parties contenant une composition selon l'une quelconque des revendications 1 à 11 et une composition contenant au moins un composé à CH acide et/ou au moins un composé contenant un groupe amino primaire ou secondaire choisi parmi le groupe qui est formé par des amines aromatiques primaires ou secondaires, des composés hétérocycliques azotés et des composés hydroxylés aromatiques, les compositions étant confectionnées à l'état réciproquement séparé dans un récipient à chambres séparées, ou respectivement dans un récipient indépendant.
